# EUROPEAN PATENT APPLICATION

(11) **EP 1 394 531 A1**
(43) Date of publication of application: **03.03.2004**
(21) Application number: 02720573.1
(22) Date of filing: 23.04.2002
(51) Int. Cl.: G01N 21/35, G01J 3/36, G01J 3/45

(54) **HANDY INTERNAL QUALITY INSPECTION INSTRUMENT**

(30) Priority: 25.04.2001 JP 2001128098
(71) Applicant: Maeda, Hiromu, Hamakita-shi, Shizuoka 434-0045 (JP); Mizuno, Toshihiro, Hamamatsu-shi, Shizuoka 431-3122 (JP)
(72) Inventor: Maeda, Hiromu, Hamakita-shi, Shizuoka 434-0045 (JP); Mizuno, Toshihiro, Hamamatsu-shi, Shizuoka 431-3122 (JP)
(74) Representative: Leson, Thomas Johannes Alois, Dipl.-Ing.
(86) International application number: PCT/JP2002/004035
(87) International publication number: WO 2002/088681

(57) **Abstract**

A compact handy type inspection instrument is provided for conducting readily nondestructive inspection of an inspection object in any working site. The inspection instrument comprises:
a spectroscope assembly containing
an optical fiber-arranging member for arranging and holding
a light-outputting end of an optical fiber bundle to be flat in a uniform layer thickness,
a packaged compact spectroscope which is enclosed in a package having a slit-shaped light inlet window on a side confronting the rectilinear light-outputting end of the optical fiber-arranging member and is constituted of a linear type continuous variable interference filter, a microlens array, and a linear type silicon array sensor assembled in the named order from the side of the light inlet window toward the opposite side, and
a positioning means for positioning the rectilinear light-outputting end of the optical fiber bundle to fit to the light input window; and a detection head; incorporated together into a main body casing.

## Description

### TECHNICAL FIELD

The present invention relates to an inspection instrument for nondestructive inspection of an object for an interior quality, an ingredient content, or the like which is not evaluatable from external appearance, the inspection object including agricultural products such as fruits, vegetables, and plant leaves; fish; meat; and other foods. In particular, the present invention relates to a compact handy type inspection instrument which enables nondestructive inspection of an object at any working site by operation of the inspection instrument by a single hand.

### BACKGROUND ART

For nondestructive inspection of interior quality, a sugar content, or the like of vegetables and fruits, an interior quality inspection instruments are used which inspect (or measure) the interior component by projecting a light beam to the vegetable or fruit and measuring the light reflected or transmitted therefrom.

Among the known examples of the inspection of components of vegetables and fruits or the like by measuring the light reflected at the interior of the vegetable or fruit, JP-A-3-160344 ("JP-A" herein means Japanese Patent Publication of unexamined application) discloses a measurement instrument for vegetable-fruit components. Another known example, JP-A-11-183374, discloses an optical sensor unit for measuring a sugar content in a vegetable or a fruit by projecting light to the vegetable or fruit and measuring the reflected light.

The aforementioned vegetable-fruit component-measurement instrument shown in JP-A-3-160344 is roughly constituted (as shown in Fig. 9) of a measurement head 2 and a main instrument part 9 (tentative name): the measurement head 2 comprising a light projector and a condenser lens (not shown in the drawing) to be brought close to a vegetable or fruit 1; the main instrument part 9 comprising a spectroscope 5 having a mirror 3 and a concave diffraction grating 4, a detector array 6 having detector elements for receiving light fractions of different wavelength bands separated by the spectroscope 5, a light-receiving element 7 for receiving reference light, and an arithmetic processing unit 8 for measuring the vegetable-fruit component by receiving electric signals generated by the detector array and the light-receiving elements. The separately constituted measurement head 2 and the main instrument part 9 are optically connected by an optical fiber 10.

The optical sensor instrument disclosed in JP-A-11-183374 is constituted, as shown in Fig. 10, of a main sensor unit 12 comprising a counterposing part 11 to be counterposed to a measurement object, and a main instrument part (tentative name) 17 comprising a concave diffraction grating 13 as a spectroscope, a photoelectric converter 14, a data processing unit 15, etc. incorporated in a casing 16. The sensor unit 12 and the main instrument part 17 are optically connected by an optical fiber 18.

As described above, in the component measurement instrument disclosed in the former publication (JP-A-3-160344), the light reflected by a vegetable or fruit and caught by the measurement head 2 is transmitted through the optical fiber 10 to the main instrument part 9, and is separated into lights of plural different wavelength bands by a concave diffraction grating 4 provided in the main instrument part 9. In the optical sensor instrument disclosed in the latter publication (JP-A-11-183374), the reflected light caught by the sensor unit 12 is transmitted to the main instrument part 17, and is separated into plural wavelength bands of light by the concave diffraction grating 13 as the spectroscope provided in the main instrument part 17.

Each of the spectroscopes described in the above patent publications employs a concave diffraction grating in the assemblage. Therefore, the main instrument part 9 or the main instrument part 17 is necessarily large in size. Further, the spectroscope having a concave diffraction grating therein should be adjusted for precise optical axis position in fixation of the aforementioned concave diffraction grating, which requires great labor and time in fixation of the concave diffraction grating to result in a higher production cost of the spectroscope.

The spectroscope having a built-in concave diffraction grating should be precisely adjusted in the alignment of the optical axes between the concave diffraction grating and the object at the light-receiving side and between the concave diffraction grating and the object at the reflection side, and further the optical axes are liable to be deviated by action of external impact or the like owing to the respective certain spatial intervals between the light outlet of the optical fiber, the reflection mirror, the concave diffraction grating, and the photoelectric converter, whereby the spectroscope is insufficient in mechanical strength and persistency disadvantageously.

The precision of spectroscopic separation can vary depending on fine displacement of the constituting members owing to thermal expansion or contraction caused by environmental temperature variation undesirably. Therefore, the conventional spectroscopes are insufficient in impact resistance (vibration resistance), so that the entire measurement instrument employing the spectroscope cannot readily be made portable (transportable), and are set usually in a testing room where the environmental temperature variation is less.

When a component or quality, for example, of a growing vegetable or fruit is inspected outdoor in a farm site without picking the vegetable or fruit by means of the aforementioned known measurement instrument or an optical sensor instrument, the measurement head 2 or the sensor unit 12 connected through the optical fiber 10 or optical fiber 18 to the main measurement instrument part 9 or the main instrument part 17 should be carried to the outdoor measurement site together with the main measurement instrument part 9 or the main instrument part 17, which causes inconvenience for handling of the main measurement instrument part 9 or the main instrument part 17.

In conducting the component inspection or quality inspection of a vegetable or a fruit in an outdoor site such as a fruit farm, preferably the inspection object is held with one hand of the inspection operator and a measurement instrument is held with the other hand, and the measurement instrument is handled by one hand with the measurement head brought into contact with the inspection object for efficiency of the inspection operation. However, in the prior art techniques as described above, the measurement head 2 and the main instrument part 9 are separated from each other, or the sensor unit 12 and the main instrument part 17 are separated from each other. With such an instrument, it is difficult to handle the measurement head 2 or the main instrument part 9 by carrying the sensor unit 12 or the main instrument part 17, which makes difficult the efficient inspection operation.

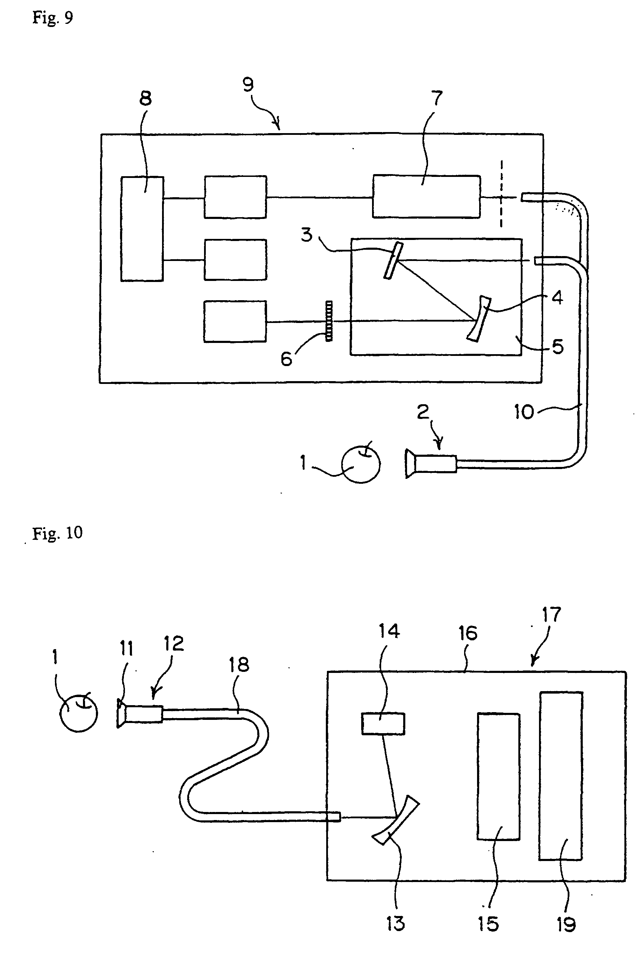

Further, in using a conventional instrument having a measurement data-indicating device 19 enclosed in the main instrument part 17 as shown in Fig. 10, the sensor unit 12 and the main instrument part having the measurement data-indicating device 19 are used separately. This makes difficult the visual confirmation of the indicated data on the indicating device 19, decreasing efficiency of the operation of measurement or inspection.

A portable optical saccharometer is disclosed in JP-A-9-89767. This known saccharometer is constituted to be portable, as shown in Fig. 11, comprising a light projecting-receiving member 21 provided on a side face of a casing 20, a light source 22 provided in the casing 20, a projecting light guide 23 for guiding the light from the light source 22 to the light projecting-receiving member 21 and to a measurement object 1 placed in front of the light projecting-receiving member 21, a received light guide 25 for guiding the light received by the light projecting-receiving member 21 to a light-separating unit 24, a data processing unit 26, and a battery not shown in the drawing; all being encased in the casing 20 to be portable.

This portable optical saccharometer, although it is small and compact, employs a concave diffraction grating in the light-separating unit 24 similarly as in the aforementioned known inspection instrument, so that the size reduction and weight reduction of the instrument is limited naturally.

This known saccharometer employs also a spectroscope which has certain spatial intervals between the outlet of transmitted light from the optical fiber, the reflection mirror, the concave diffraction grating, and the photoelectric converter, respectively. Therefore, the light reflection tends to vary depending on thermal distortion or displacement of the constituting members caused by environmental temperature or other factors at the outdoor inspection site, causing deviation in the relation between the photoelectric output and the wavelength to lower the measurement precision, disadvantageously.

### DISCLOSURE OF THE INVENTION

The present invention has been made in view of the above situations. The present invention intends to provide a handy type interior quality inspection instrument which employs a packaged compact spectroscope in place of a conventional spectroscopic means employing a reflection mirror and a concave diffraction grating requiring a certain spatial interval and is useful for nondestructive inspection of interior qualities by spectroscopic analysis of inspection objects (agricultural products such as fruits, vegetables, and plant leaves; fish; meat; and other foodstuffs). This instrument is capable of inspecting (measuring) interior quality of an inspection object with high precision with high mechanical strength and high shock resistance of the instrument without influence of environmental temperature and other conditions.

The present invention intends also to provide a light-weight compact handy type interior quality inspection instrument which enables easy inspection of an interior quality of objects, even if the object is a growing fruit on a tree before harvest in an orchard, by holding the inspection instrument with one hand, or which enables inspection of an object by holding the inspection instrument with one hand in any inspection site in any posture of the inspection operator.

The present invention intends further to provide a handy type interior quality inspection instrument which enables instant checking of inspection results at the inspection operation site by reading the displayed inspection data or measurement data at the site of inspection of the interior quality of the inspection object.

The present invention intends still further to provide a handy type interior quality inspection instrument which comprises a connector for outputting the measurement data of many inspection objects to enable analysis of the measurement data of inspection objects such as fruits at a central control room or a like place to obtain cultivation control data to investigate the dependence of the interior quality of the inspection object on the cultivation environment, fertilizer, and the like.

To attain the above object, in claim 1 of the present invention, a handy type interior quality inspection instrument is provided which comprises a detection head having a light-emitting unit for projecting light to an inspection object and a light-receiving unit for receiving light diffuse-reflected and transmitted from interior of the inspection object, a spectroscope for receiving the transmitted light from the detection head through an optical fiber bundle and separating spectroscopically the received light into light fractions, a photoelectric converter for converting the fractions of light separated by the spectroscope into electric signals, and a data processing unit for processing the electric signals transmitted from the photoelectric converter,
wherein the interior quality inspection instrument comprises:
a spectroscope assembly containing
an optical fiber-arranging member for arranging and holding a light-outputting end of the optical fiber bundle to be flat in a uniform layer thickness to keep the light-outputting end in a rectilinear shape,
a packaged compact spectroscope which is enclosed in a package having a slit-shaped light inlet window on a side confronting the face of the rectilinear light-outputting end of the optical fiber-arranging member and is constituted of a linear type continuous variable interference filter and a linear type silicon array sensor assembled in the named order from the side of the light inlet window toward the opposite side, and
a positioning means for positioning the rectilinear light-outputting end of the optical fiber bundle to fit to the light input window; a power source battery; and
a detection head; incorporated together into a main body casing.

In claim 2 of the present invention, to attain the above object, in the handy type interior quality inspection instrument of claim 1, a grip portion which is handleable with a single hand is provided in integration with the main body casing, and an indicating means is provided which has an operation switch knob manipulatable from outside the main body case.

In claim 3 of the present invention, to attain the above object, in the handy type interior quality inspection instrument of claim 1 or 2, an indicating means is provided which has an indicator face on an outside of the main body case.

In claim 4 of the present invention, to attain the above object, in the handy type interior quality inspection instrument of claim 1, 2, or 3, a communication connector is provided, on the main body case, for transmitting data of inspection results obtained by arithmetic processing in the data processing unit to an external instrument.

In claim 5 of the present invention, in the handy type interior quality inspection instrument of claim 1, the optical fiber bundle is formed by knitting a number of micro-optical fibers at random, and the light-inputting end thereof is positioned in a light introduction hole of the detection head, and the light-outputting end is positioned to fit to a light input window of the packaged compact spectroscope.

In claim 6 of the present invention, in the handy type interior quality inspection instrument of claim 1, the linear type continuous variable interference filter allows the light to pass in the range from a short wavelength of 600 nm to a long wavelength of 1100 nm and separates selectively the filtered light spectroscopically to vary linearly with the wavelength.

In claim 7 of the present invention, in the handy type interior quality inspection instrument of claim 1, the packaged compact spectroscope comprises, in the package, a linear type continuous variable interference filter, a microlens array, and a linear type silicon array sensor assembled in the named order from the light inlet window of the package toward the opposite side, and the microlens array is constituted of a number of microlenses arranged in juxtaposition to focus fractions of the light separated by the linear type continuous variable interference filter respectively onto light-receiving faces of the elements of the linear type silicon array sensor.

In claim 8 of the present invention, in the handy type interior quality inspection instrument of claim 2 or 3, the indicating means is an LED indicator which indicates measured values for respective measurement items such as a sugar content, and acidity by selection by an operation switch.

In claim 9 of the present invention, in the handy type interior quality inspection instrument of claim 2 or 8, the indicating means is a liquid crystal indicator for indicating measurement items and corresponding measured values of such as a sugar content and acidity.

In claim 10 of the present invention, in the handy type interior quality inspection instrument of claim 2, 3, 8, or 9, wherein the indicating means is provided on an outside of the main body casing.

In claim 11 of the present invention, in the handy type interior quality inspection instrument of claim 2, the grip portion is formed to protrude out in a T-shape from the main body casing, and a trigger type operation switch is provided near the grip.

In claim 12 of the present invention, in the handy type interior quality inspection instrument of claim 2 or 11, the grip portion is provided in integration in one body with the circuit-encasing chamber in which the axis of the grip and the axis of the circuit-encasing chamber are parallel to each other.

In claim 13 of the present invention, in the handy type interior quality inspection instrument of claim 2, 11, or 12, a battery-encasing room is provided within the grip for encasing a power-supplying battery.

In claim 14 of the present invention, the handy type interior quality inspection instrument of claim 1, the detection head has a flexible padding member at the top end for close contact with an inspection object at periphery of the padding member with a prescribed contact area, the light projection hole of the padding member is surrounded by a protruding edge, and the spatial interval between the light inlet hole and the periphery of the padding member is designed to be larger than the spatial interval between the light projection hole and the light reception hole.

As described above, the handy type interior quality inspection instrument of the present invention having the aforementioned construction employs a packaged compact spectroscope comprising a linear type continuous variable interference filter and a linear type silicon array sensor without employing a diffraction grating. By use of the packaged compact spectroscope, the handy type interior quality inspection instrument is made compact and lightweight. This compact inspection instrument can be handled by a single hand just like the handling of a pistol, so that the inspection can be conducted in any posture of the inspection operator with the inspection object such as a growing fruit before harvest on a tree or those after the harvest.

In the inspection instrument of the present invention, the output end of the optical fiber bundle and the packaged compact spectroscope are positioned fixedly by a housing box as the positioning means. Thereby, the light is transmitted from the light output end of the optical fiber bundle into the packaged compact spectroscope, and is allowed to pass through the solid of the linear type continuous variable interference filter to be separated into light fractions. The light fractions reach the linear type silicon array sensor without diffusion. Therefore, the output corresponding to the respective wavelength fractions can be taken out with high precision to enable measurement with high inspection precision without deviation of the optical axis independently of variation of the environmental conditions such as temperature.

By encasing the power source battery within the grip portion provided in integration with the main instrument body casing, the gravity center of the inspection instrument is brought near to the joint portion between the grip portion and the main body case, which facilitates handling of the instrument by a single hand. Thereby the detection head of the instrument can be positioned precisely to face the inspection object.

The pad provided at the tip of the detection head is flexible. Therefore the pad can be attached, with the periphery thereof, to the inspection object with a prescribed contact area (contact width). The interval (distance) between the periphery of the pad and the light-receiving face of the optical fiber bundle is larger than the interval (distance) between the light-receiving face of the fiber group and the small lamp. Therefore, only the light projected from the small lamp and diffuse-reflected from the inspection object is introduced to the optical fiber bundle without influence of diffuse-reflection light caused by external disturbing light.

The indicating device is provided on the main body casing to indicate the measurement data and the like of the inspection results. This makes easier the reading of the measurement data (inspection results) in consideration of the state of contact of the detection head with the inspection object (influence of light leakage or external disturbing light), and so forth.

### BRIEF DESCRIPTION OF THE DRAWINGS

Fig. 1 is a side view of an example of the interior quality inspection instrument of the present invention with the principal portions broken away.
Fig. 2 is a front view of the interior quality inspection instrument of the present invention.
Fig. 3 is a rear view of the interior quality inspection instrument of the present invention.
Fig. 4 is a sectional plan view of the interior quality inspection instrument of the present invention with the principal portion broken away.
Fig. 5 explains a state of using the interior quality inspection instrument of the present invention.
Fig. 6 is a drawing for explaining a detailed construction of the interior quality inspection instrument of the present invention with the principal portions broken away.
Fig. 7 is a side view of another example of the interior quality inspection instrument of the present invention.
Fig. 8 is a side view of still another example of the interior quality inspection instrument of the present invention.
Fig. 9 is a circuit diagram for explaining a conventional interior quality inspection instrument.
Fig. 10 is a circuit diagram for explaining another conventional interior quality inspection instrument.

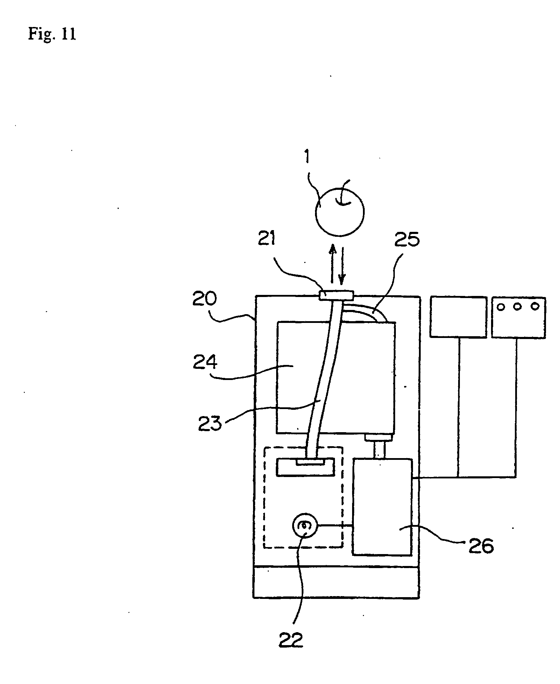

Fig. 11 is a diagram for explaining still another conventional interior quality inspection instrument.
Fig. 12 is an enlarged diagram of a principal part for explaining another example of use of the interior quality inspection instrument of the present invention.

### BEST MODE FOR CARRYING OUT THE INVENTION

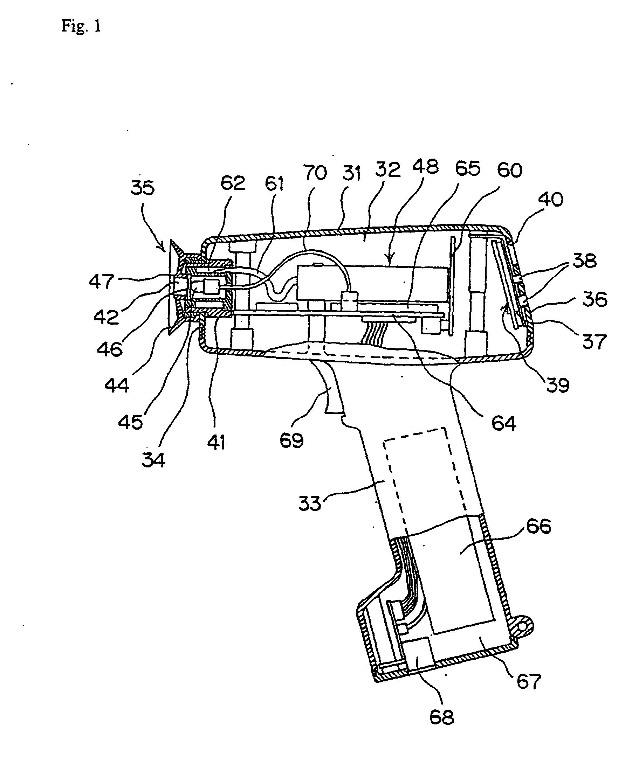

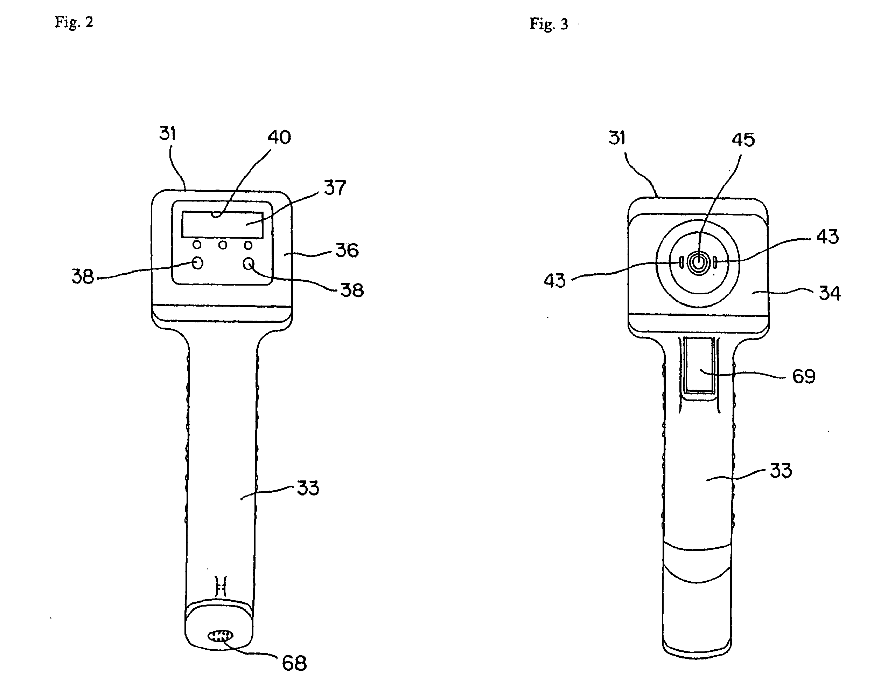

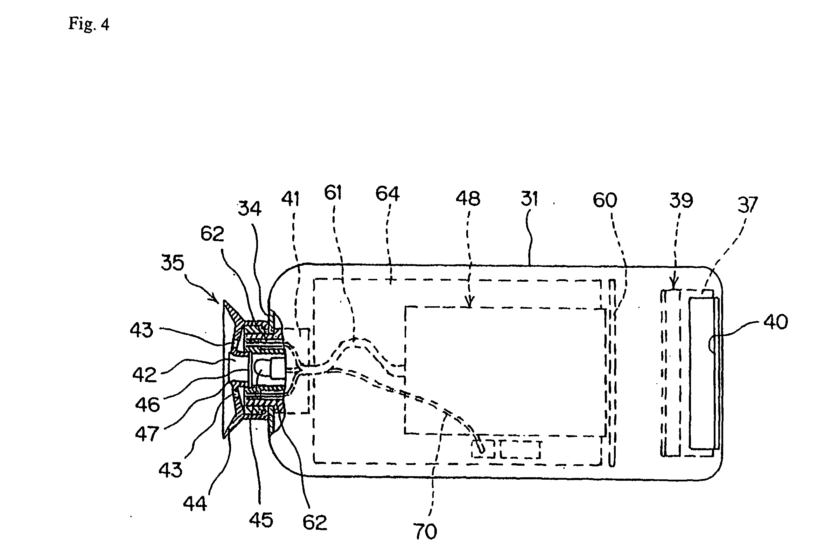

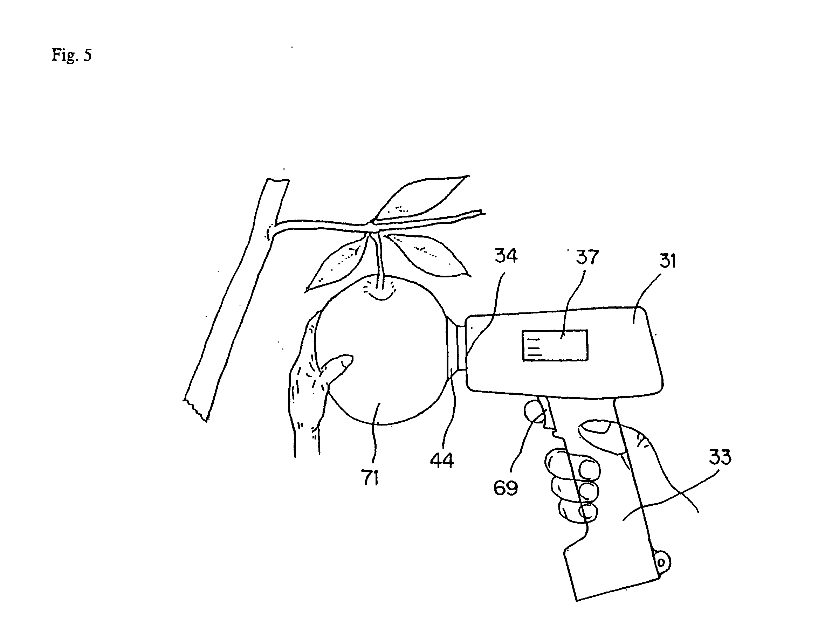

A first example of the present invention is explained below in detail by reference to Figs. 1-6. Fig. 1 is a side view with the principal part broken away, Fig. 2 is a front view, Fig. 3 is a rear view, Fig. 4 is an enlarged plan view with the detection head broken away, and Figs. 5-6 are drawings for explanation of a state of the inspection operation.

The numeral 31 indicates a main body casing. The main body casing 31 is formed in integration of a circuit-encasing chamber 32 directed laterally and a grip 33 directed slantingly. A detection head 35 penetrates into the circuit-encasing chamber 32 to be held by a front wall 34 of the circuit-encasing chamber 32. An indicating means 39 having an indicator 37 comprising an LED or LCD and an operating switch knob 38 is fitted to the inside of a rear wall 36 of the circuit-encasing chamber 32. The indication by the indicator 37 of indicating means 39 can be read visually through an indication window 40 on the rear wall 36. The respective operating switch knobs 38 of the indicating means 39 can be handled by a finger of the hand holding the grip 33 from outside the main body casing 31.

The detection head 35 comprises a head case 41 fixed by penetration through the front wall 34 of the circuit-encasing chamber 32; a padding member 44 fitted to the outside of the front end of the head case 41, having a light projection hole 42 and light reception holes 43 at the both side of the light projection hole 42, and formed of a flexible elastic material like a rubber or an elastomer; and a small lamp 45 placed at the center of the head case 41 for projecting light outside through the light projection hole 42.

This small lamp 45 is preferably a halogen lamp which emits light in the range from visible light to infrared light, but is not limited to thereto, and any light source may be used which emits light ranging from visible light to infrared light. In front of the small lamp 45, a transparent glass plate 46 is provided for dust protection. Around the light projection hole 42, a protruding aperture-edge 47 is provided to intercept light leakage to the light reception hole 43 and to improve the contact with the inspection object.

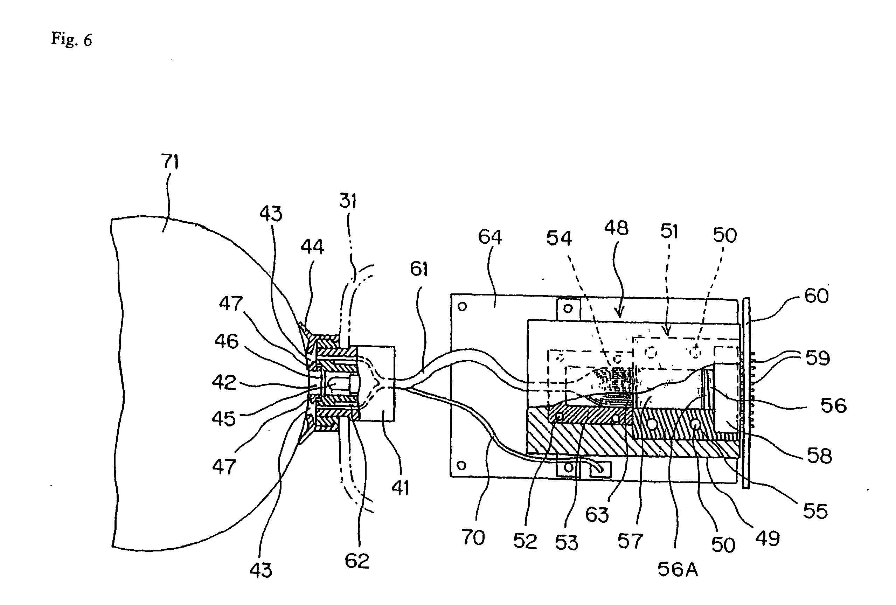

The numeral 48 denotes a spectroscope assembly fixed in the circuit-encasing chamber 32. This spectroscope assembly 48, as shown in Fig. 6, is constituted of a packaged compact spectroscope 51 and an optical fiber arraying member 53 in integration: the packaged compact spectroscope 51 being fixed in a housing box 49 through plural fixing means 50 to the housing box 49 as a positioning means, and the optical fiber-arraying member 53 being fixed in a housing box 49 similarly through plural fixing means 52 to the housing box 49 as a positioning means without relative positional deviation from the packaged compact spectroscope 51. The packaged compact spectroscope 51 is assembled in a package 55 having a light inlet window 54, comprising a linear type continuous variable interference filter 56, and a linear type silicon array sensor 58 arranged integrally in this order from the light inlet window 54 toward the opposite side. Sensor output terminals 59 protruding from the linear type silicon array sensor outside the package 55 are connected to a sensor circuit substrate 60. The linear type continuous variable interference filter 56 and the linear type silicon array sensor may be placed adjacently, or placed with interposition of a microlens array 57.

The linear type continuous variable interference filter 56 allows the light to pass in the range from a short wavelength of 600 nm to a long wavelength of 1100 nm, and separates the light with linear variation of the wavelength selectively. This linear type continuous variable interference filter 56 is in a thin film state for filtration of the light with the film thickness controlled to vary from the short wavelength end to the long wavelength end.

The microlens array 57, when it is provided, is placed in a line or lines between the linear type continuous variable interference filter 56 and a linear type silicon array sensor 58 placed in opposition at a prescribed distance. The microlens array is constituted of many microlenses to focus the light fractions separated spectroscopically by the linear type continuous variable interference filter 56 respectively on the light-receiving elements (chips) of the linear type silicon array sensor 58 for the respective wave lengths without diffusion of light.

The array may be arranged in zigzag or in lines in accordance with the arrangement of the light-receiving elements of the linear type silicon array sensor 58.

An optical fiber bundle 61 is formed by knitting many optical micro-fibers. The light input end of the optical fiber bundle 61 is divided into plural bundles (two bundles in this example), and the ends of the divided respective bundles are placed through grooves 62 of the head case 41 of the detection head 35 to receive the light from the light-reception holes 43 of a padding member 44. The light input ends may be combined into one.

The light output end 63 of the optical fiber bundle 61 is broadened in a plate shape and arranged at random in a brush or a flat brush in a uniform layer thickness of, for example, about 1 mm or less. The fiber ends are arranged and held in a rectilinear shape in the fiber arranging member 53 which arranges and holds the light output ends to fit the shape of the light inlet window 54 formed on the linear type continuous variable interference filter.

The optical fiber arranging member 53 for arranging and holding the light output end 63 of the optical fiber bundle 61 is positioned in a housing box 49 as the positioning means in such a manner that the light output end 63 of the optical fiber bundle 61 fits to the position of the light inlet window 54 of the packaged compact spectroscope 51. Thus the spectroscope assembly 48 is constituted.

The numeral 64 denotes a main circuit substrate having thereon an arithmetic processing circuit 65 which conducts spectroscopic analysis based on the output from the linear type silicone array sensor 58. The size of the main circuit substrate 64 is preferably smaller than the size of a palm for the handy type of the instrument. Such a small size of the main circuit substrate enables a small size of the main body casing 31.

Inside the grip 33, a battery-encasing room 67 is provided for encasing a battery 66 (e.g., a rechargeable battery). At the end of the grip 33, a communication connector 68 is provided for communication with an external processing means.

Outside the grip 33, a pistol lever type operation switch 69 is provided. The numeral 70 denotes an electric power supply line for the small lamp 45. The numeral 71 denotes a fruit as the inspection object.

Next, the function of the interior quality inspection instrument of the above constitution is described below. The handy type interior quality inspection instrument of the above constitution can be made compact in its entirety owing to compactness of the spectroscope assembly 48 built in the main body casing 31. This compact interior quality inspection instrument can be of a compact single-handy type manipulatable by a single hand.

With this interior quality inspection instrument, a fruit 71 growing on a tree, for example, can be inspected as it is for the interior quality. As shown in Fig. 5, a padding member 44 of the detection head 35 of the interior quality inspection instrument is pushed against the surface of the fruit 71 to bring the periphery of the padding member 44 and an aperture edge 47 into close contact with the fruit 71. Then the small lamp is turned on by pulling the operation switch 69 by a forefinger of the operator to project light through the light projection hole 42 into the fruit 71.

The light projected into the fruit 71, is diffuse-reflected in the interior of fruit 71 as the inspection object in accordance with the interior quality. The diffuse-reflected light is introduced from the light introduction hole 43 through the optical fiber bundle 61 into the spectroscope assembly 48. The light introduced into the spectroscope assembly 48 is guided from the light outlet end of the optical fiber bundle arranged in a shape of a rectilinear flat plate into the packaged compact spectroscope 51 containing the linear type continuous variable interference filter 56 and the linear type silicon array sensor 58. By this packaged compact spectroscope 51, respective different wavelengths of light are transformed into electric signals.

The electric signals outputted from the packaged compact spectroscope 51 are introduced to arithmetic processing circuit 65 to be processed in a conventional manner for the inspection (measurement) of the intended sugar content, acidity, or the like properties of the fruit, and the inspection results are shown by the indicator 37.

The inspection data or other data can be transmitted through the communication connecter 68 to a personal computer or the like equipped in a control room to prepare control data.

In the above examples, the indicator 37 is provided on the rear face of the main body casing 31 in Figs. 1-4, and is provided on the side face of the main body casing 31 in Fig. 5. However, the position of the indicator 37 is not limited thereto, and may be provided on the upper face of the main body casing 31.

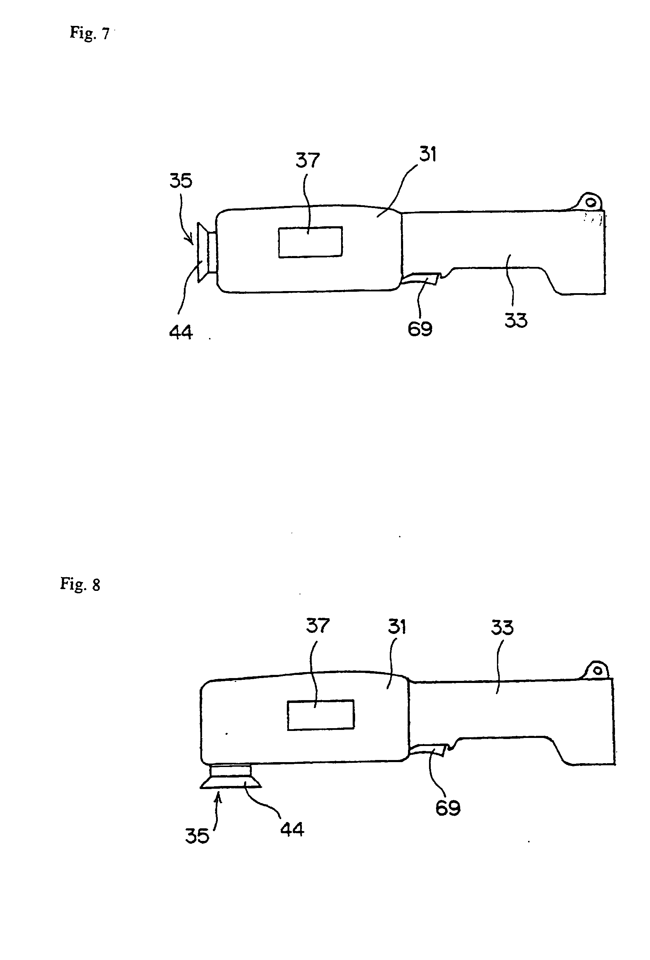

In the above example, the grip 33 is of a pistol type. In a second example shown in Fig. 7, the main body casing 31 is in a stick shape with the axis of the circuit enclosing case 32 and the axis of the grip 33 being directed in the same direction.

In a third example, a detection head 35 directed downward is provided on a lower face of a main body case 31 having the same shape as that of the second example shown in Fig. 7. The setting direction and shape of the detection head 35 are not limited specially insofar as the main body casing 31 is a handy type one which is handleable by a single hand.

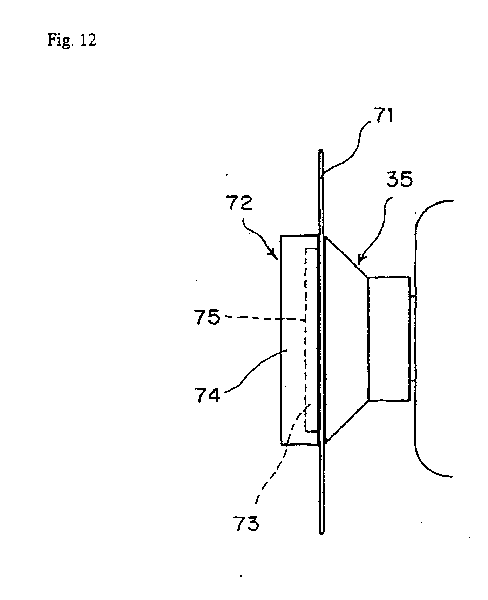

Fig. 12 shows a state of practice of inspection of interior quality of a plant leaf by means of the interior quality inspection instrument of the present invention.

An inspection object 71 which is thin like a plant leaf is not inspectable for chlorophyll or the like in the leaf by the aforementioned interior quality inspection instrument since the light projected from the detection head 35 to the leaf permeates through the interior of the leaf. Such a thin inspection object 71 like a plant leaf can be inspected for the interior quality by placing a light reflection plate 72 on the reverse side of the inspection object.

The light reflection plate 72 is preferably constituted of a mirror capable of back face reflection. For example, as shown in Fig. 12, the mirror is a back-face reflection plate which is constituted of a transparent glass plate 73 having a reflection face 75 formed by mercury vapor deposition or a like method on the backside and a cover 74 for holding the reflective plate, or is a back-face reflection plate 72 which is constituted of a transparent glass plate 73 and a cover 74 having a reflective face formed by mercury deposition or a like method on the face contacting with the transparent glass plate 73. With such a back-face reflection plate 72, the light having transmitted through the thin inspection object 71 is reflected toward the detection head.

As described above, the interior quality inspection instrument set forth in claim 1 of the present invention is constituted such that the diffuse-reflected light transmitted from the inside of the inspection object through the output end of an optical fiber bundle is spectroscopically separated directly by a linear type continuous variable interference filter and the separated light fractions are converted by a linear type silicon array sensor into spectroscopic output for respective wavelengths. Thus the transmitted light is not affected by environmental variation such as temperature variation since the light is transmitted through the solid matters only. Accordingly, in the present invention, the measurement results or inspection results are obtained stably without the disadvantages of variation or deviation of the measured values or inspected values by environmental variation such as temperature, differently from conventional interior quality inspection instruments employing a reflection mirror or a concave diffraction grating.

The packaged compact spectroscope, which employs combination of a linear type continuous variable interference filter and a linear type silicon array sensor, enables the portable or transportable lightweight structure of the interior quality inspection instrument in the present invention. Thereby, the interior component can be measured or inspected of an inspection object like fruits growing on a tree before the harvest in a fruit farm in any posture of the inspection operator.

According to the invention set forth in claim 2 of the present invention, the interior quality inspection instrument has a main body case and the grip integrated in one body for single hand manipulation of the instrument. Thereby, the inspection can be conducted by holding the inspection instrument of the present invention with one hand just as holding a pistol or the like and holding the inspection object with the other hand in an arbitrary posture of the inspection operator, whereby the inspection can be conducted with significantly improved inspection working efficiency.

According to the invention set forth in claim 3, the inspection data or the like can be instantly confirmed during the inspection operation at the inspection site. Therefore, the present invention can be applied suitably to the inspection with observation of relations to growing conditions such as sunshine and fertilizer.

According to the invention set forth in claim 4, the data communication function is provided for transmitting the measurement data (inspection data) or the like stored in the memory on the substrate to a personal computer or other external control mechanism to obtain statistical control data to utilize effectively the information for quality control.

According to the invention set forth in claim 14, a power source battery is housed in the grip portion, whereby the gravity center is brought to the grip portion. Therefore, the detection head can be held steadily in close contact with the inspection object, and the inspection operation can be conducted in that contacting state without hand waving by keeping the contacting posture, resulting in high inspection accuracy.

### INDUSTRIAL APPLICABILITY

The handy type interior quality inspection instrument of the present invention is compact and handy, being useful for inspecting nondestructively an inspection object for interior property (interior quality or component) which is not detectable simply from external appearance: the inspection object including agricultural products such as fruits, vegetables, and plant leaves; fish; meat; and other foodstuffs.

## Claims

1. A handy type interior quality inspection instrument comprising a detection head having a light-emitting unit for projecting light to an inspection object and a light-receiving unit for receiving light diffuse-reflected and transmitted from interior of the inspection object, a spectroscope for receiving the transmitted light from the detection head through an optical fiber bundle and separating spectroscopically the received light into light fractions, a photoelectric converter for converting the fractions of light separated by the spectroscope into electric signals, and a data processing unit for processing the electric signals transmitted from the photoelectric converter,
wherein the interior quality inspection instrument comprises:
a spectroscope assembly containing
an optical fiber-arranging member for arranging and holding a light-outputting end of the optical fiber bundle to be flat in a uniform layer thickness to keep the face of the light-outputting end in a rectilinear shape,
a packaged compact spectroscope which is enclosed in a package having a slit-shaped light inlet window on a side confronting the rectilinear light-outputting end of the optical fiber-arranging member and is constituted of a linear type continuous variable interference filter and a linear type silicon array sensor assembled in the named order from the side of the light inlet window toward the opposite side, and a positioning means for positioning the rectilinear light-outputting end of the optical fiber bundle to fit to the light input window;
a power source battery; and
a detection head;
incorporated together into a main body casing.

2. The handy type interior quality inspection instrument according to claim 1, wherein a grip portion which is handleable with a single hand is provided in integration with the main body casing, and an indicating means is provided which has an operation switch knob manipulatable from outside the main body case.

3. The handy type interior quality inspection instrument according to claim 1 or 2, wherein an indicating means is provided which has an indicator face on an outside of the main body case.

4. The handy type interior quality inspection instrument according to claim 1, 2, or 3, wherein a communication connector is provided, on the main body case, for transmitting data of inspection results obtained by arithmetic processing in the data processing unit to an external instrument.

5. The handy type interior quality inspection instrument according to claim 1, wherein the optical fiber bundle is formed by knitting a number of micro-optical fibers at random, and the light-inputting end thereof is positioned in a light introduction hole of the detection head, and the light-outputting end thereof is positioned to fit to a light input window of the packaged compact spectroscope.

6. The handy type interior quality inspection instrument according to claim 1, wherein the linear type continuous variable interference filter allows the light to pass in the range from a short wavelength of 600 nm to a long wavelength of 1100 nm and separates selectively the filtered light spectroscopically to vary linearly with the wavelength.

7. The handy type interior quality inspection instrument according to claim 1, wherein the packaged compact spectroscope comprises, in the package, a linear type continuous variable interference filter, a microlens array, and a linear type silicon array sensor assembled in the named order from the light inlet window of the package toward the opposite side, and the microlens array is constituted of a number of microlenses arranged in juxtaposition to focus fractions of the light separated by the linear type continuous variable interference filter respectively onto light-receiving faces of the elements of the linear type silicon array sensor.

8. The handy type interior quality inspection instrument according to claim 2 or 3, wherein the indicating means is an LED indicator which indicates measured values for respective measurement items such as a sugar content, and acidity by selection by an operation switch.

9. The handy type interior quality inspection instrument according to claim 2 or 8, wherein the indicating means is a liquid crystal indicator for indicating measurement items and corresponding measured values of such as a sugar content and acidity.

10. The handy type interior quality inspection instrument according to claim 2, 3, 8, or 9, wherein the indicating means is provided on an outside of the main body casing.

11. The handy type interior quality inspection instrument according to claim 2, wherein the grip portion is formed to protrude out in a T-shape from the main body casing, and a trigger type operation switch is provided near the grip.

12. The handy type interior quality inspection instrument according to claim 2 or 11, wherein the grip portion is provided in integration in one body with the circuit-encasing chamber in which the axis of the grip and the axis of the circuit-encasing chamber are parallel to each other.

13. The handy type interior quality inspection instrument according to claim 2, 11, or 12, wherein a battery-encasing room is provided within the grip for encasing a power-supplying battery.

14. The handy type interior quality inspection instrument according to claim 1, wherein the detection head has a flexible padding member at the top end for close contact with an inspection object at periphery of the padding member with a prescribed contact area, the light projection hole of the padding member is surrounded by a protruding edge, and the special interval between the light inlet hole and the periphery of the padding member is designed to be larger than the spatial interval between the light projection hole and the light reception hole.
